# EUROPEAN PATENT APPLICATION

(11) **EP 1 886 709 A1**
(43) Date of publication of application: **13.02.2008**
(21) Application number: 07114099.0
(22) Date of filing: 09.08.2007
(51) Int. Cl.: A61M 25/06

(54) **Indwelling catheter, hollow needle, and indwelling needle assembly**

(30) Priority: 11.08.2006 JP 2006220427
(71) Applicant: Medikit Co., Ltd., Tokyo, 113-0034 (JP)
(72) Inventor: Nakajima, Hiroaki, Tokyo (JP)
(74) Representative: Kreutzer, Ulrich

(57) **Abstract**

An indwelling catheter comprises a catheter tube (4). The catheter tube is provided with an inserted portion which is to be inserted into a patient and a main portion which is provided on a base end (5) side of the inserted portion. Furthermore, a tapered portion is formed on at least a part of the inserted portion. An outer diameter of the inserted portion is thinner than an outer diameter of the main portion. According to the above-mentioned indwelling catheter, since the outer diameter of the inserted portion is made thinner than that of the main portion by providing the tapered portion, a pricking pain of a patient is reduced. In addition, since the outer diameter of the inserted portion is made thin without the need of making the outer diameter of the main portion thin, a sufficient flow amount of fluid for medical interventions can be secured.

## Description

### BACKGROUND OF THE INVENTION

### 1. Field of the Invention

The present invention relates to an indwelling catheter, a hollow needle, and an indwelling needle assembly used for hemodialysis, medical solution or blood infusion, and so on.

### 2. Description of the Related Art

Hemodialysis, medical solution or blood infusion, and so on are done in medical interventions. Indwelling needles are used for these medical procedures. The indwelling needle has a flexible outer tube (= an indwelling catheter) made of Teflon (registered trademark), polyurethane, or the like, and a metal syringe needle inserted into the outer tube. The syringe needle is removed from the outer tube after inserting the indwelling needle into a blood vessel. Then, the outer tube is indwelled to infuse medical solution or blood into the blood vessel.

Generally, the indwelling needle has a double-tube structure composed of a catheter tube and an inner needle. Upon its use, the indwelling needle is inserted into the blood vessel with the inner needle having been inserted into the catheter tube. The inner needle is removed when the indwelling needle has been inserted enough to the appropriate position. Then, a tube for infusion is connected to a base body of the catheter tube and medical solution or blood is infused into the blood vessel via the catheter tube. For example, this kind of the indwelling needle is disclosed in Laid-open Japanese Patent Application No. 2002-126090.

### SUMMARY OF THE INVENTION

However, with the above-mentioned indwelling needle, a person (hereinafter, he/she is called "patient"), into whom the indwelling needle is inserted, may feel a sharp pricking pain when the indwelling needle is inserted, because the inserted portions of the inner needle and the catheter is thick.

It is conceivable that the inner needle and the catheter should be made thinner to relieve the pain. But it results in that securing of a sufficient flow amount for the medical interventions becomes difficult.

The present invention is intended for providing an indwelling catheter, a hollow needle, and an indwelling needle assembly which can relieve a pain of insertion by making the inserted portion thin and can secure a sufficient flow amount for the medical interventions.

A first aspect of the present invention provides an indwelling catheter which comprises a catheter tube. The catheter tube is provided with an inserted portion which is to be inserted into a patient and a main portion which is provided on a base end side of the inserted portion. A tapered portion is formed on at least a part of the inserted portion. An outer diameter of the inserted portion is thinner than an outer diameter of the main portion.

It is preferable that the tapered portion has a smooth stream-lined shape.

It is also preferable that the main portion has at least one side hole.

It is also preferable that the indwelling catheter further comprises: a catheter base which is connected to a base end of the catheter tube; a flexible clamp tube which is connected to a base end of the catheter base; and a luer connector which is connected to a base end of the clamp tube.

A second aspect of the present invention provides an indwelling needle assembly which comprises: an indwelling catheter which has all the features of the above-mentioned first aspect of the present invention; and a hollow needle set into the indwelling catheter.

It is preferable that the indwelling needle assembly further comprises a hemostatic valve attached to a base end of the luer connector.

A third aspect of the present invention provides a hollow needle which comprises: an inserted distal portion which is to be inserted into a patient; and a hollowed main portion which is provided on a base end side of the inserted distal portion. An outer diameter of the inserted distal portion is thinner than an outer diameter of the hollowed main portion.

It is preferable that the hollow needle further comprises a tapered portion which is formed on at least a part of the inserted distal portion and the tapered portion has a smooth stream-lined shape.

It is also preferable that the hollowed main portion has at least one side hole.

A fourth aspect of the present invention provides an indwelling needle assembly which comprises: an inner needle which is to be inserted into a patient; and an indwelling catheter into which the inner needle is inserted to be set. The indwelling catheter is provided with a catheter tube which is made of transparent material. The catheter tube is provided with an inserted portion which is to be inserted into the patient along with the inner needle and a main portion which is provided on a base end side of the inserted portion. A tapered portion is formed on at least a part of the inserted portion. An outer diameter of the inserted portion is thinner than an outer diameter of the main portion. The inner needle is provided with an inserted distal portion which is made of metal and a hollowed main portion which is made of transparent material.

It is preferable that the tapered portion has a smooth stream-lined shape.

It is also preferable that the main portion has at least one side hole.

It is also preferable that the indwelling catheter further comprises: a catheter base which is connected to a base end of the catheter tube; a flexible clamp tube which is connected to a base end of the catheter base; and a luer connector which is connected to a base end of the clamp tube.

It is also preferable that the indwelling catheter further comprises a hemostatic valve attached to a base end of the luer connector.

### BRIEF DESCRIPTION OF THE DRAWINGS

- Fig. 1: is a cross sectional view of an indwelling needle assembly according to a first embodiment of the present invention;
- Fig. 2: is a side view of a catheter of the indwelling needle assembly according to the first embodiment of the present invention;
- Fig. 3: is a close-up side view of a tip end of the catheter of the indwelling needle according to the first embodiment of the present invention;
- Fig. 4A: is a close-up side view of another tip end of a catheter of the indwelling needle according to the first embodiment of the present invention;
- Fig. 4B: is a close-up side view of another tip end of a catheter of the indwelling needle according to the first embodiment of the present invention;
- Fig. 5A: is a close-up plan view of a tip end of a hollow needle according to a second embodiment of the present invention;
- Fig. 5B: is a close-up bottom view of the tip end of the hollow needle according to the second embodiment of the present invention;
- Fig. 6: is a plan view of a tip end of an inner needle of an indwelling needle assembly according to a third embodiment of the present invention; and
- Fig. 7: is a side view of the indwelling needle assembly according to the third embodiment of the present invention showing a visual state of blood.

### DETAILED DESCRIPTION OF PREFERRED EMBODIMENTS

### < First embodiment >

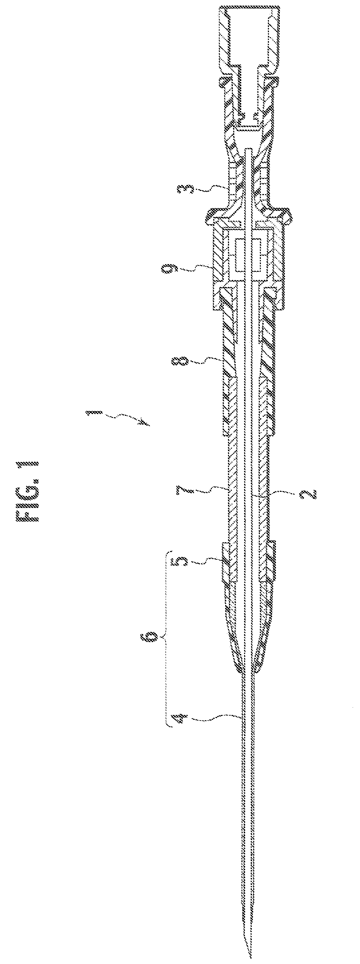

A first embodiment of the present invention will be described hereinafter with reference to drawings. Fig. 1 is a cross sectional view showing a structure of an indwelling needle assembly 1 having an indwelling catheter 6 according to the first embodiment.

As shown in Fig. 1, the indwelling needle assembly 1 is provided with an inner needle (hollow needle) 2 which is to be inserted into a patient, an inner needle base 3 holding a base end of the inner needle 2, a catheter 6 provided with a catheter tube 4 and a catheter base 5, a clamp tube 7 connected to the catheter 6, a connector (luer connector) 8, and a hemostatic valve 9.

The inner needle 2 is a hollow needle which is inserted into a lumen of a catheter tube 4. The inner needle 2 has an outer diameter which is slightly smaller than an inner diameter of the catheter tube 4 to be able to be inserted thereinto. The inner needle 2 is made of stainless steel, aluminum, titanium, composition metal of these metal materials, or the like. Since the inner needle 2 is to be inserted into the patient while being inserted within the catheter tube 4, the inner needle 2 has a sufficient length so that its tip end can be projected out from the tip end of the catheter tube 4. The inner needle 2 has a sharp blade edge on its tip end. This sharp blade edge has an inclined blade plane in order to reduce an inserting resistance.

The inner needle base 3 is attached to the base end of the inner needle 2. The inner needle base 3 is made of transparent or semi-transparent toughened plastics. A person who inserts the indwelling needle assembly 1 into the patient inserts the catheter 6 into the patient while holding the inner needle base 3. After inserting the catheter 6 into the patient, he/she removes the inner needle 2 by pulling out the inner needle base 3 under holding the clamp tube 7. The hemostatic valve 9 prevents leak of blood when the inner needle 2 is removed.

The above-mentioned structure of the indwelling needle assembly 1, except the catheter 6, is disclosed as a sample. The above-mentioned structure does not limit the structure of the present invention.

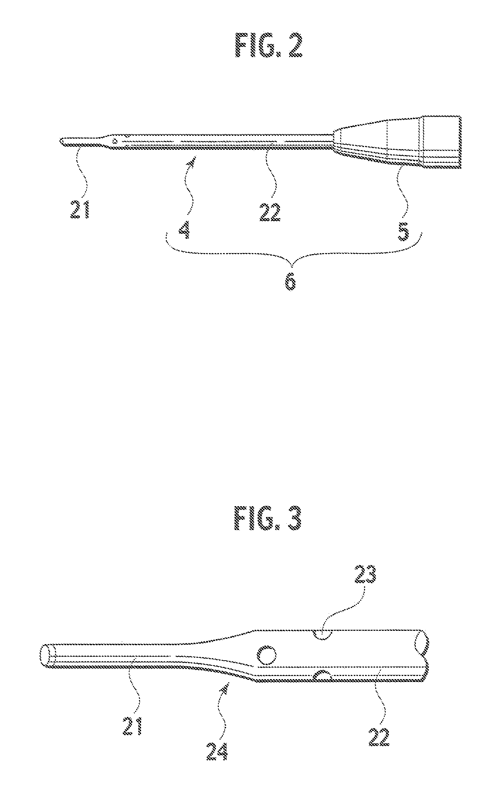

Next, a structure of the catheter 6 will be described with reference to Figs. 2 and 3. As shown in Fig. 2, the catheter 6 is provided with the catheter tube 4 into which the inner needle 2 is inserted and the catheter base 5 to which a base end of the catheter tube 4 is attached.

As shown in the close-up view of Fig. 3, the catheter tube 4 has a tapered portion 24 on a part of an inserted portion 21 which is to be inserted into the patient. Thus, the inserted portion 21 is thinner in an outer diameter than a main portion 22 of the catheter tube 4. For example, the outer diameter of the inserted portion 21 is made as 20 G (Gauge) and that of the main portion 22 is made as 18 G.

Since the outer diameter of the tip end of the inserted portion 21 has almost the same outer diameter of the inner needle 2 and the outer diameter becomes gradually thicker as it approaches to the main portion 22, a pricking pain of the patient is reduced by reducing the inserting resistance of the inserted portion 21. Furthermore, since the outer diameter of the main portion 22 does not need to be made thin, a sufficient flow amount of fluid flowing through the catheter tube 4 for the medical interventions can be secured.

The tapered portion 24 which is formed on a base end side of the inserted portion 21 has a smooth stream-lined shape (it is also called "bullet shape" or "cone shape", especially, Fig. 1 (or 3) shows a smooth "concave" cone shape) and then the outer diameter of the catheter tube 4 is made gradually thinner as it approaches to the tip end. Thus, the pricking pain of the patient can be reduced by reducing the inserting resistance of the inserted portion 21.

Furthermore, as shown in Fig. 3, the main portion 22 of the catheter tube 4 has a plurality of side holes 23. The flow amount of fluid flowing through the catheter tube 4 can be regulated by adjusting the number of the side holes 23 and each inner diameter of the side holes 23. Therefore, the sufficient flow amount for the medical interventions can be secured by selecting a specific catheter 6 that has the adequate number of the side holes 23 with the adequate inner diameter.

The indwelling catheter assembly 1 constituted as mentioned above is inserted into the blood vessel of the patient with the inner needle 2 having been inserted within the catheter 6. The inner needle 2 and the inner needle base 3 are removed when the catheter tube 4 has been inserted enough to the appropriate position. Then, the hemostatic valve 9 is also removed. After the catheter tube 4 has been indwelled in a body of the patient, an infusion tube is connected to the connector 8 for infusing medical solution or blood into the blood vessel through the catheter tube 4.

Therefore, the catheter tube 4 is generally made of pliable plastic in order not to damage the blood vessel. And the inner needle 2 is generally made of metal in order to be inserted into the blood vessel easily.

As mentioned above, with the catheter 6 of the present embodiment, since the outer diameter of the inserted portion 21 of the catheter tube 4 is thinner than that of the main portion 22, the pricking pain of the patient can be reduced and a sufficient flow amount for the medical interventions can be secured.

In addition, with the catheter 6 of the present embodiment, since the catheter tube 4 is made gradually thinner by the smooth stream-lined shape of the tapered portion 24 on the inserted portion 21, the pricking pain of the patient can be reduced by reducing the inserting resistance.

Furthermore, with the catheter 6 of the present embodiment, since the main portion 22 of the catheter tube 4 has the side holes 23 for regulating the flow amount of fluid flowing through the catheter tube 4 by adjusting the number of the side holes 23 and the inner diameter of the side holes 23, the sufficient flow amount for the medical interventions can be secured.

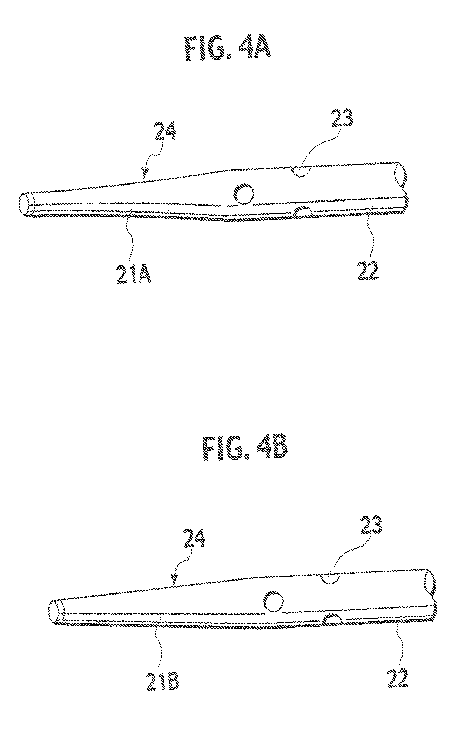

The inserted portion 21 of the catheter tube 4 is not limited to the shape shown in Fig. 3 but may have another shape. For example, as shown in an inserted portion 21A of Fig. 4A, the tapered portion 24 is formed on all range of the inserted portion 21 and has a smooth stream-lined shape (it may be also called "bullet shape" or "cone shape", especially, Fig. 4A shows a smooth "concave" cone shape) overall. Then, the diameter of the catheter tube 4 is made gradually thinner as it approaches to the tip end within the whole range of the inserted portion 21A. Otherwise, as shown in an inserted portion 21 B of Fig. 4B, the tapered portion 24 is formed on all range of the inserted portion 21 and has a gently cone shape (especially, smooth "flat" cone shape) overall. Then the diameter of the catheter tube 4 is made gradually thinner as it approaches to the tip end within the whole range of the inserted portion 21A. The tapered portion 24 may have a smooth convex cone shape.

In these manners, the tapered portion 24 may be formed not only on a part of the inserted portion 21 as shown in Fig. 3 but also on the whole range of the inserted portion 21 as shown in Figs. 4A and 4B. In addition, the shape of the tapered portion 24 is preferably formed as a smooth stream-lined shape (it is also called "bullet shape" or "cone shape"). It may be formed as a gently cone shape, a body-of-revolution shape which has a gently inclined surface, or a shape of composition of these shapes.

### < Second embodiment >

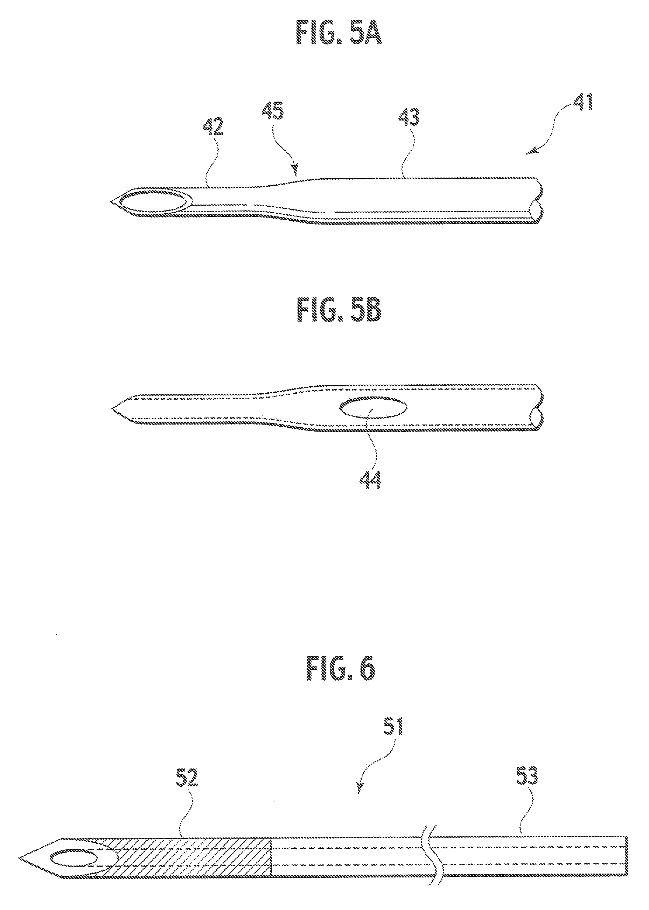

A second embodiment of the present invention will be described hereinafter with reference to drawings. Figs. 5A and 5B are cross sectional views showing a structure of a hollow needle 41 according to the second embodiment.

As shown in Fig. 5A, the hollow needle 41 is made of stainless steel, aluminum, titanium, composition metal of these metal materials, or the like. The hollow needle 41 has a hollow tubular structure and its inner hollow space is formed from a tip end to a base end.

Then, an inserted distal portion 42 which is to be inserted into the patient is thinner in an outer diameter than a hollowed main portion 43. For example, the outer diameter of the inserted distal portion 42 is made as 19 G and that of the hollowed main portion 43 is made as 16 G.

Thus, since an inserting resistance of the inserted distal portion 42 is reduced by making the inserted distal portion 42 thinner than the hollowed main portion 43, a pricking pain of the patient is reduced. Furthermore, since an outer diameter of the hollowed main portion 43 does not need to be made thin, a sufficient flow amount of fluid for the medical interventions can be secured.

A tapered portion 45 which is formed on a base end side of the inserted distal portion 42 has a smooth stream-lined shape (it is also called "bullet shape" or "cone shape", especially, Fig. 5A or 5B shows a smooth "concave" cone shape) and the outer diameter of the hollow needle 41 is made gradually thinner as it approaches to the tip end. Thus, the pricking pain of the patient can be reduced by reducing the inserting resistance of the inserted distal portion 42. The tapered portion 45 may have a smooth flat cone shape or a smooth convex cone shape.

Furthermore, as shown in Fig. 5B, the hollowed main 25 portion 43 has a side hole 44. The flow amount of fluid flowing through the hollow needle 41 can be regulated by adjusting the number of the side holes 44 and an inner diameter of the side hole 44. Therefore, the sufficient flow amount for the medical interventions can be secured by selecting a specific hollow needle 41 that has the adequate number of the side holes 44 with the adequate inner diameter.

In the above-mentioned first embodiment, the inserted portion 21 of the catheter tube 4 is made gradually thinner 5 as it approaches to the tip end. In the present embodiment, the hollow needle 41 is also made gradually thinner as it approaches to the tip end. Thus, the hollow needle 41 of the present embodiment may be used with a conventional catheter (instead of the catheter 6 of the first embodiment). By using the hollow needle 41 with a conventional catheter, the same effect as with the first embodiment (reducing the pain and securing the sufficient flow amount) can be achieved with the hollow needle 41 of the present invention.

### < Third embodiment >

A third embodiment of the present invention will be described hereinafter with reference to drawings. Figs. 6 is a plan view of a tip end of an inner needle 51 of an indwelling needle assembly according to the third embodiment. Here, the indwelling needle assembly of the present embodiment, except the inner needle 51, has the same structure as the indwelling needle assembly 1 of the first embodiment (Figs. 1 to 3, 4A and 4B). Therefore, the description about the elements except for the inner needle 51 is omitted hereinbelow.

As shown Fig. 6, in the present embodiment, an inner needle 51 of the indwelling assembly is provided with an inserted distal portion 52 made of metal and a needle main portion 53 made of transparent material such as plastics, or the like. Since it is made of stainless steel, aluminum, titanium, composition metal of these metal materials, or the like, the inserted distal portion 52 is easy to be inserted into a blood vessel.

Since it is made of the transparent or semi-transparent toughened material such as polystyrene, polyethylene, polypropylene, or the like, the needle main portion 53 enables to make a back-flow of blood visible.

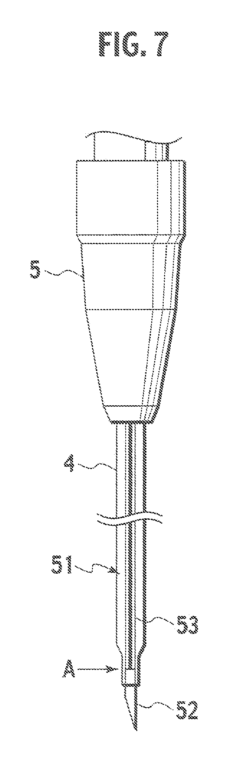

In a conventional indwelling needle assembly, a position where a back-flow of blood can be visible is the inner needle base 3 provided at the base end of the inner needle 2 (see Fig. 1 as an alternative reference of the conventional indwelling needle assembly). On the other hand, with the inner needle 51 of the present embodiment, a back-flow of blood can be visible just after the back-flow has passed a connecting position A of the inserted distal portion 52 and the needle main portion 53, as shown in Fig. 7.

As mentioned above, with the indwelling needle assembly with the inner needle 51 of the present invention, a back-flow of blood is easy to be confirmed, since the inserted distal portion 52 is made of metal and the needle main portion 53 is made of transparent material, the back-flow of blood can be found immediately.

According to the indwelling catheter, the hollow needle, or the indwelling needle assembly of the present invention, since an outer diameter of an inserted portion is made thinner than that of a main portion by providing a tapered portion on at least a part of the inserted portion of a catheter tube, a pricking pain of a patient is reduced. Furthermore, since an outer diameter of the inserted portion is made thin without the need for making an outer diameter of the main portion thin, a sufficient flow amount of fluid for medical interventions can be secured.

Although the invention has been described above by reference to certain embodiments of the invention, the invention is not limited to the embodiments described above. Modifications and variations of the embodiments described above will occur to those skilled in the art, in light of the above teachings.

The contents of Japanese Patent Application No. 2006-220427 (filed August 11, 2006) are incorporated herein by reference in its entirety.

## Claims

1. An indwelling catheter (6) comprising a catheter tube (4), wherein
- the catheter tube is provided with an inserted portion (21) which is to be inserted into a patient and a main portion (22) which is provided on a base end side of the inserted portion,
- a tapered portion (24) is formed on at least a part of the inserted portion, and
- an outer diameter of the inserted portion is thinner than an outer diameter of the main portion.

2. The indwelling catheter according to claim 1, wherein the tapered portion has a smooth stream-lined shape.

3. The indwelling catheter according to Claim 1 or 2, wherein the main portion has at least one side hole (23).

4. The indwelling catheter according to any one of claims 1 to 3, further comprising:
- a catheter base (5) which is connected to a base end of the catheter tube;
- a flexible clamp tube (7) which is connected to a base end of the catheter base; and
- a luer connector (8) which is connected to a base end of the clamp tube.

5. A hollow needle (41) comprising:
- an inserted distal portion (42) which is to be inserted into a patient; and
- a hollowed main portion (43) which is provided on a base end side of the inserted distal portion,
- wherein an outer diameter of the inserted distal portion is thinner than an outer diameter of the hollowed main portion.

6. The hollow needle according to claim 5, further comprising
- a tapered portion (45) which is formed on at least a part of the inserted distal portion,
- wherein the tapered portion has a smooth stream-lined shape.

7. The hollow needle according to claim 5 or 6, wherein the hollowed main portion has at least one side hole (44).

8. An indwelling needle assembly (1) comprising:
- a hollow inner needle (2; 51) which is to be inserted into a patient and
- an indwelling catheter (6) according to one of the claims 1 - 4 into which the inner needle is inserted to be set.

9. The indwelling needle assembly (1) according to claim 8,
wherein said indwelling catheter comprises a
- a catheter base (5) which is connected to a base end of the catheter tube;
- a flexible clamp tube (7) which is connected to a base end of the catheter base; and
- a luer connector (8) which is connected to a base end of the clamp tube;
and wherein the indwelling needle assembly further comprises
- a hemostatic valve (9) attached to a base end of the luer connector.

10. The indwelling needle assembly (1) according to claim 8 or 9, wherein said catheter tube (4) is made of transparent material.

11. The indwelling needle assembly (1) according to one of the claims 8 - 10, wherein said hollow inner needle is provided with an inserted distal portion (52) which is made of metal and a hollowed main portion (53) which is made of transparent material.
